# EUROPEAN PATENT APPLICATION

(11) **EP 4 693 320 A1**
(43) Date of publication of application: **11.02.2026**
(21) Application number: 24780508.8
(22) Date of filing: 27.03.2024
(51) Int. Cl.: G16H 20/00, G06Q 10/04

(54) **METHOD FOR CREATING BODY CONDITION VARIATION PREDICTION MODEL, BODY CONDITION VARIATION PREDICTION SYSTEM, BODY CONDITION VARIATION PREDICTION METHOD, BODY CONDITION VARIATION PREDICTION MODEL, AND METHOD FOR CREATING REGULARITY IDENTIFICATION MODEL**

(30) Priority: 31.03.2023 JP 2023059622
(71) Applicant: Minebea Mitsumi Inc., Kitasaku-gun, Nagano 3890293 (JP)
(72) Inventor: FUSE Tohru, Kitasaku-gun, Nagano 389-0293 (JP); ZAITSU Yusuke, Kitasaku-gun, Nagano 389-0293 (JP)
(74) Representative: Winter, Brandl - Partnerschaft mbB
(86) International application number: PCT/JP2024/012365
(87) International publication number: WO 2024/204406

(57) **Abstract**

A method for creating a physical condition variation prediction model (M_{PRE}) for predicting variation in a physical condition of a subject (S) on a bed (BD) includes acquiring biological information of a subject for teaching data by a sensor (LS1 to LS4), determining a bed rest state of the subject for teaching data on the basis of the biological information of the subject for teaching data, acquiring a state trend corresponding to a trend of the bed rest state per unit time, identifying a regularity included in temporal fluctuation of the state trend, and creating, by supervised machine learning using teaching data obtained by associating the regularity and physical condition variation information indicating variation in a physical condition of the subject for teaching data, a physical condition variation prediction model obtained by learning a correlation between the regularity and the physical condition variation information of the subject for teaching data.

## Description

### Technical Field

The present invention relates to a method for creating a physical condition variation prediction model, a physical condition variation prediction system, a physical condition variation prediction method, a physical condition variation prediction model, and a method for creating a regularity identification model.

### Background Art

In medical and caregiving settings, acquiring data from a subject on a bed by a sensor and predicting a change in a physical condition of the subject on the basis of the acquired data has been proposed.

Patent Document 1 discloses a physical condition prediction method of acquiring body movement data of a target person, continually determining a sleep state of the target person on the basis of the acquired body movement data, and predicting a change in a physical condition of the target person on the basis of the determined sleep state.

### Citation List

### Patent Literature

Patent Document 1: JP 2019-76689 A

### Summary of Invention

### Technical Problem

In technology for predicting a physical condition change in a subject, further improvement in prediction accuracy is desired, including in the physical condition prediction method disclosed in Patent Document 1.

An object of the present invention is to provide a method for creating a physical condition variation prediction model capable of creating a physical condition variation prediction model with improved prediction accuracy.

An object of the present invention is to provide a physical condition variation prediction system, a physical condition variation prediction method, and a physical condition variation prediction model with improved prediction accuracy.

An object of the present invention is to provide a method for creating a regularity identification model, the method being for creating a regularity identification model used in a physical condition variation prediction system and a physical condition variation prediction method with improved prediction accuracy.

### Solution to Problem

According to a first aspect of the present invention, provided is a method for creating a physical condition variation prediction model for predicting variation in a physical condition of a subject on a bed. The method includes acquiring biological information of a subject for teaching data by a sensor, determining a bed rest state of the subject for teaching data on the basis of the biological information of the subject for teaching data, acquiring a state trend corresponding to a trend of the bed rest state per unit time, identifying a regularity included in temporal fluctuation of the state trend, and creating, by supervised machine learning using teaching data obtained by associating the regularity and physical condition variation information indicating variation in a physical condition of the subject for teaching data, a physical condition variation prediction model obtained by learning a correlation between the regularity and the physical condition variation information of the subject for teaching data.

According to a second aspect of the present invention, provided is a physical condition variation prediction system for predicting variation in a physical condition of a subject on a bed. The physical condition variation prediction system includes a bed rest state determination unit configured to determine a bed rest state of the subject on the basis of biological information of the subject acquired by a sensor, a state trend acquisition unit configured to acquire a state trend corresponding to a trend of the bed rest state per unit time, a regularity identification unit configured to identify a regularity included in temporal fluctuation of the state trend, and a physical condition variation prediction unit configured to input the regularity into a physical condition variation prediction model to predict variation in a physical condition of the subject. The physical condition variation prediction model is a learned model obtained by learning, by supervised machine learning using teaching data obtained by associating the regularity identified for a subject for teaching data and physical condition variation information indicating variation in a physical condition of the subject for teaching data, a correlation between the regularity and the physical condition variation information of the subject for teaching data.

According to a third aspect of the present invention, provided is physical condition variation prediction method for predicting variation in a physical condition of a subject on a bed. The physical condition variation prediction method includes determining a bed rest state of the subject on the basis of biological information of the subject acquired by a sensor, acquiring a state trend corresponding to a trend of the bed rest state per unit time, identifying a regularity included in temporal fluctuation of the state trend, and inputting the regularity into a physical condition variation prediction model to predict variation in a physical condition of the subject. The physical condition variation prediction model is a learned model obtained by learning, by supervised machine learning using teaching data obtained by associating the regularity identified for a subject for teaching data and physical condition variation information indicating variation in a physical condition of the subject for teaching data, a correlation between the regularity and the physical condition variation information of the subject for teaching data.

According to a fourth aspect of the present invention, provided is a physical condition variation prediction model for predicting variation in a physical condition of a subject on a bed. By supervised machine learning using teaching data created by acquiring biological information of a subject for teaching data by a sensor, determining a bed rest state of the subject for teaching data on the basis of the biological information of the subject for teaching data, acquiring a state trend corresponding to a trend of the bed rest state per unit time, identifying a regularity included in temporal fluctuation of the state trend, and associating the regularity and physical condition variation information indicating variation in a physical condition of the subject for teaching data, a correlation between the regularity and the physical condition variation information of the subject for teaching data is learned. Variation in a physical condition of the subject is predicted on the basis of the regularity.

According to a fifth aspect of the present invention, provided is a method for creating a regularity identification model. The method includes acquiring biological information of a subject on a bed by a sensor, determining a bed rest state of the subject on the basis of the biological information of the subject, acquiring a state trend corresponding to a trend of the bed rest state per unit time, and creating, by machine learning using the state trend as training data, a regularity identification model configured to identify a predetermined regularity included in temporal fluctuation of the state trend.

### Advantageous Effects of Invention

According to the method for creating a physical condition variation prediction model of the present invention, it is possible to create a physical condition variation prediction model with improved prediction accuracy.

According to the physical condition variation prediction system, the physical condition variation prediction method, and the physical condition variation prediction model of the present invention, it is possible to predict variation in a physical condition of a subject with improved prediction accuracy.

According to the method for creating a regularity identification model of the present invention, it is possible to create a regularity identification model used in a physical condition variation prediction system and a physical condition variation prediction method with improved prediction accuracy.

### Brief Description of Drawings

FIG. 1 is a block diagram illustrating a configuration of a physical condition variation prediction system.
FIG. 2 is an explanatory diagram illustrating an arrangement of load sensors with respect to a bed.
FIG. 3 is a flowchart illustrating steps of a physical condition variation prediction method executed by the physical condition variation prediction system.
FIG. 4 is a diagram conceptually illustrating a flow of processing in the physical condition variation prediction method executed by the physical condition variation prediction system.
FIG. 5 is a graph showing an example of a frequency spectrum obtained on the basis of a load signal.
FIG. 6 is a graph showing a bed rest state of a subject in time series.
FIG. 7 is a graph showing a state trend of the subject in time series.
FIG. 8 is a flowchart illustrating steps of a method for creating a bed rest state determination model.
FIG. 9 is a graph showing an example of temporal fluctuation of four load signals detected by four load sensors.
FIG. 10 is a flowchart illustrating steps of a method for creating a regularity identification model.
FIG. 11 is a flowchart illustrating steps of a method for creating a physical condition variation prediction model.

### Description of Embodiments

### Embodiments

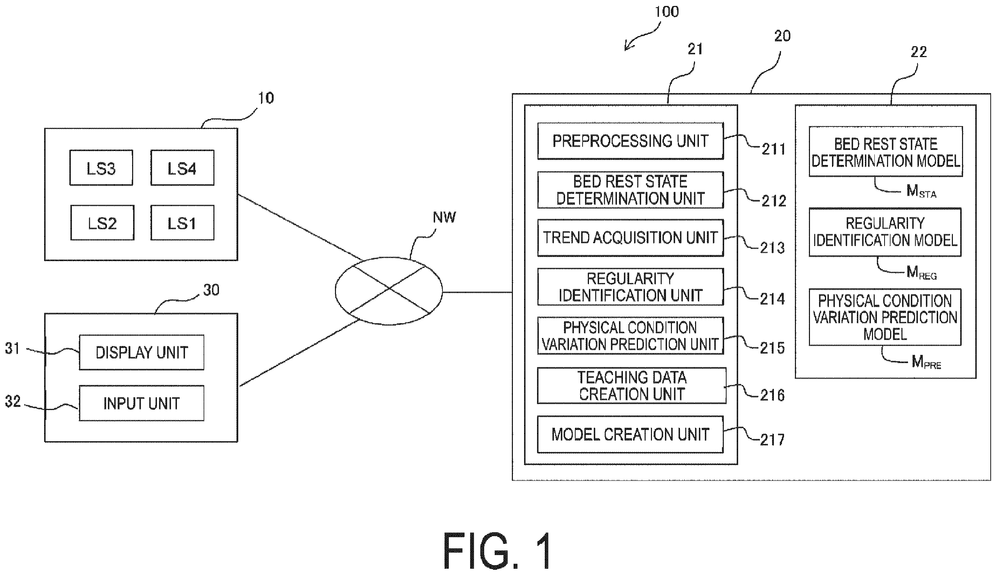

A physical condition variation prediction system 100 and a physical condition variation prediction method according to an embodiment of the present invention will now be described with reference to FIG. 1 to FIG. 7.

### Physical Condition Variation Prediction System

As illustrated in FIG. 1, the physical condition variation prediction system 100 mainly includes a load measurement unit 10, an information processing device 20, and a terminal device 30. The load measurement unit 10, the information processing device 20, and the terminal device 30 are connected to one another via a network NW such as the Internet. Note that at least two of the load measurement unit 10, the information processing device 20, and the terminal device 30 may be connected by a wiring line.

The load measurement unit 10 mainly includes four load sensors LS1, LS2, LS3, and LS4. Each of the four load sensors LS1 to LS4 measures a load using a load cell having a beam shape, for example. Such a load sensor is described in, for example, JP 4829020 B and JP 4002905 B.

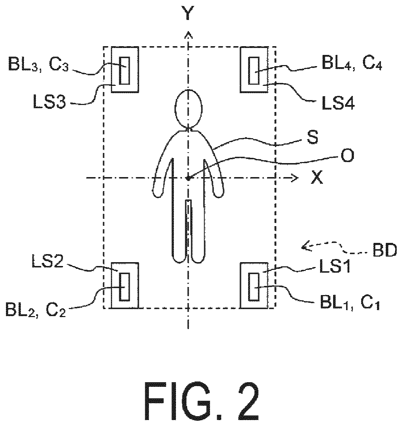

As illustrated in FIG. 2, the four load sensors LS1 to LS4 are respectively disposed under casters C₁, C₂, C₃, and C₄ attached to lower end parts of legs BL₁, BL₂, BL₃, and BL₄, at four corners of a bed BD used by a subject S.

The information processing device 20 mainly includes a control unit 21 and a storage unit 22. A preprocessing unit 211, a bed rest state determination unit 212, a trend acquisition unit 213, a regularity identification unit 214, a physical condition variation prediction unit 215, a teaching data creation unit 216, and a model creation unit 217 are constructed in the control unit 21 (details described below). The storage unit 22 stores a bed rest state determination model M_{STA}, a regularity identification model M_{REG}, and a physical condition variation prediction model M_{PRE} (details described below).

The information processing device 20 as well as the control unit 21 and the storage unit 22 inside the information processing device 20 may be configured in a single physical server or may be formed in a plurality of physical servers (that is, cloud servers) dispersedly and connected by a cloud.

The terminal device 30 mainly includes a display unit 31 and an input unit 32.

The display unit 31 receives a prediction of variation in a physical condition of the subject S output by the physical condition variation prediction unit 215, and displays the prediction to a user (doctor, nurse, caregiver, home caregiver, or the like) of the physical condition variation prediction system 100. The display unit 31 includes, for example, an image display unit such as a liquid crystal display, and/or a voice display unit such as a speaker.

The input unit 32 is an interface for performing various inputs to the physical condition variation prediction system 100. The input unit 32 may be, for example, a keyboard and/or a mouse, and may be a touchscreen formed integrally with a liquid crystal display of the display unit 31.

### Physical Condition Variation Prediction Method

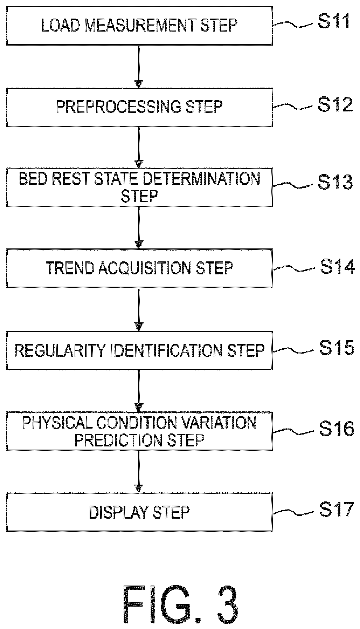

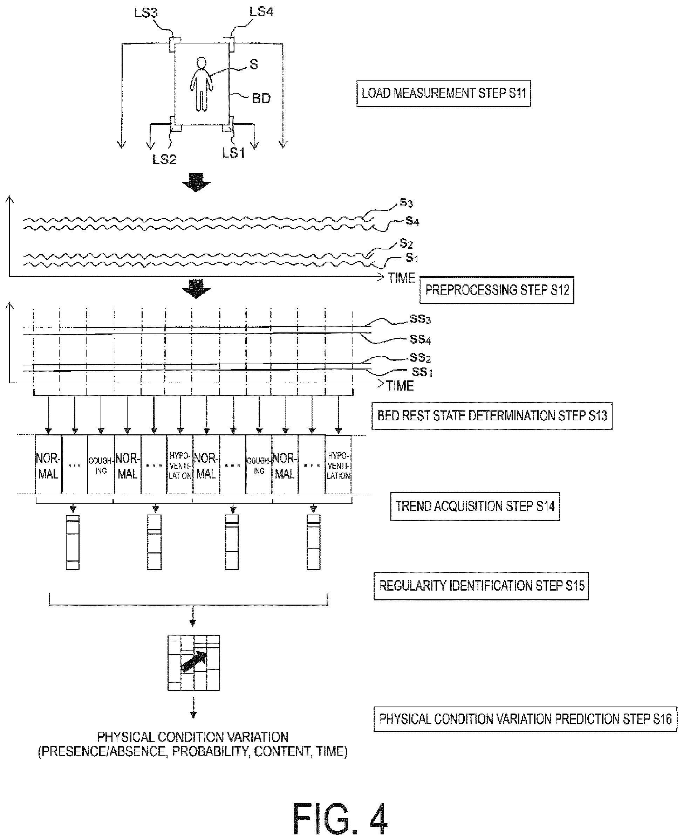

Each step of the physical condition variation prediction method executed by the physical condition variation prediction system 100 will now be described with reference to the flowchart of FIG. 3 and the conceptual diagram of FIG. 4.

As illustrated in the flowchart of FIG. 3, the physical condition variation prediction method executed by the physical condition variation prediction system 100 includes a load measurement step S11, a preprocessing step S12, a bed rest state determination step S13, a trend acquisition step S14, a regularity identification step S15, a physical condition variation prediction step S16, and a display step S17.

### Load Measurement Step S11

In the load measurement step S11, a load of the subject S on the bed BD (that is, load applied to the bed BD by the subject S) is measured using the load sensors LS1 to LS4 of the load measurement unit 10. The load of the subject S is dispersedly applied to the load sensors LS1 to LS4 disposed under the legs BL₁ to BL₄ at the four corners of the bed BD, and is dispersedly measured by the load sensors LS1 to LS4.

Each of the load sensors LS1 to LS4 detects the load of the subject S as an analog signal. Hereinafter, the analog signals (that is, raw data signals) detected by the load sensors LS1, LS2, LS3, and LS4 are described as analog signals si, s₂, s₃, and s₄.

The analog signals s₁ to s₄ are sent to the information processing device 20 via a communication interface (not illustrated) of the load measurement unit 10 and the network NW.

### Preprocessing Step S12

In the preprocessing step S12, the preprocessing unit 211 of the information processing device 20 calculates various parameters on the basis of load values of the subject S (analog signals s₁, s₂, s₃, and s₄) received from the load measurement unit 10 via the network NW.

The preprocessing unit 21 first receives the analog signals si to s₄ sent from the load measurement unit 10 via the network NW and a communication interface (not illustrated) of the information processing device 20. Then, the analog signals s₁ to s₄ are digitally converted with a sampling period of 5 milliseconds, for example, and digital signals are acquired. Hereinafter, digital signals obtained by digitally converting the analog signals s₁, s₂, s₃, and s₄ are described as load signals ssi, ss₂, ss₃, and ss₄, respectively.

In the present embodiment, the preprocessing unit 211 calculates at least one of the parameters described below. The preprocessing unit 211 stores the calculated parameters in the storage unit 22.

### (1) Load signals ssi to ss₄

The load signals ss₁ to ss₄ are directly used as the parameters.

### (2) Spectrum amplitude SAn_{X to Y} in each frequency band

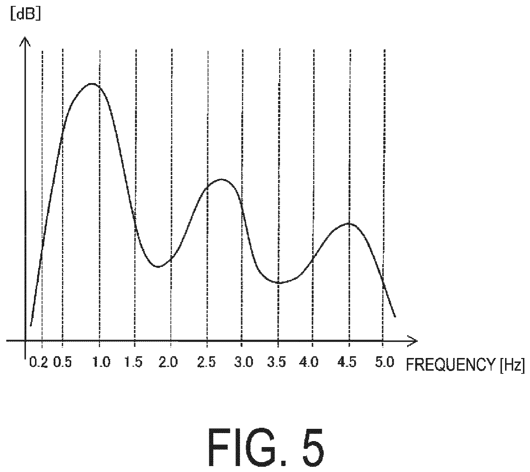

Short-time Fourier transform is sequentially performed on the load signals ss₁ to ss₄ to sequentially generate a frequency spectrum (example illustrated in FIG. 5). A length of a window function of the short-time Fourier transform can be, for example, about 10 s. The short-time Fourier transform may be executed, by way of example, every 5 s to 15 s. The window functions of time-consecutive two short-time Fourier transforms may partially overlap each other.

The length of the window function of the short-time Fourier transform is known to be correlated with time resolution and frequency resolution. That is, if the time length of the window function is lengthened, the time resolution becomes low and the frequency resolution becomes high, and if the time length of the window function is shortened, the time resolution becomes high and the frequency resolution becomes low. As a technique for improving such characteristics, suppression of side lobe by introducing the window function is known. Further, generalized harmonic analysis (GHA) and short-time Fourier transform using a plurality of window lengths (multi-windows STFT) are known. Using these techniques, the frequency spectrum may be analyzed and the explanatory variables may be determined.

For each of the generated frequency spectra, the frequency is divided into 10 bands, and a mean value of the spectrum amplitudes in each band is calculated. In the present embodiment, as an example, the mean value of the spectrum amplitudes in each of the bands of 0.2 to 0.5 Hz, 0.6 to 1.0 Hz, 1.1 to 1.5 Hz, 1.6 to 2.0 Hz, 2.1 to 2.5 Hz, 2.6 to 3.0 Hz, 3.1 to 3.5 Hz, 3.6 to 4.0 Hz, 4.1 to 4.5 Hz, and 4.6 to 5.0 Hz is calculated.

Hereinafter, the mean value of the spectrum amplitudes in the frequency band of X to Y Hz of the frequency spectrum generated on the basis of the load signal ssₙ (n = 1, 2, 3, 4) is described as a spectrum amplitude SAn_{X to Y} (n = 1, 2, 3, 4).

### (3) Position of center of gravity

This is a position of center of gravity of the subject S on the bed BD calculated on the basis of the load signals ss₁ to ss₄. Any known method can be used for calculation of the position of center of gravity. An example of a calculation method of the position of center of gravity is described in JP 6321719 B.

### (4) Respiratory waveform

A center of gravity G of the subject S vibrates in a direction along a body axis (spine) of the subject S in response to respiration of the subject S (hereinafter, this vibration is called "respiratory vibration"). The respiratory waveform indicates a respiratory vibration, and the horizontal axis represents time and the vertical axis represents amplitude of the respiratory vibration. An example of a drawing method of a respiratory waveform is described in JP 6661173 B.

### (5) Respiratory rate

The respiratory rate of the subject S is calculated on the basis of, for example, a respiratory waveform. Since one cycle of the respiratory waveform corresponds to respiration (expiration and inspiration) of one time, a respiratory rate B for 1 minute is estimated as B = 60/T, a peak to peak distance of the respiratory waveform being T.

### (6) Respiratory amplitude value

The respiratory rate of the subject S is calculated on the basis of, for example, a respiratory waveform. The amplitude of the respiratory waveform can be estimated as a respiratory amplitude value.

### (7) Heart rate waveform

As an example, a waveform obtained by separating components included in the frequency band (approximately 0.5 Hz to approximately 3.3 Hz) of the heart rate from each of the load signals ss₁ to ss₄ can be a heart rate waveform.

### (8) Activity index (ACI)

The ACI is an index indicating the activity of the subject. For example, the ACI is calculated as a time integrated value in a predetermined period (for example, 20 seconds) of a simple mean value of standard deviations σ₁ to σ₄, standard deviations of the temporal fluctuation of the load signals ssi, ss₂, ss₃, and ss₄ being σ₁, σ₂, σ₃, and σ₄, respectively. In place of the standard deviations σ₁ to σ₄, values obtained by dividing the standard deviations σ₁ to σ₄ by the amplitude of the respiratory waveform may be used. Further details of the ACI are described in JP 6661173 B.

### (9) Total load

Total load can be calculated as a sum of the load values indicated by the load signals ssi to ss₄.

### (10) Other

In addition, it is possible to create and use a predetermined parameter estimated to be related to a state in accordance with the state to be classified.

### Bed Rest State Determination Step S13

In the bed rest state determination step S13, the bed rest state determination unit 212 inputs at least one of the various parameters calculated in the preprocessing step S12 to the bed rest state determination model M_{STA} to determine the bed rest state of the subject S.

The bed rest state determination model M_{STA} is a learned model created so as to determine the bed rest state of the subject S on the bed BD on the basis of a predetermined explanatory variable (creation method described below). The bed rest state determination model M_{STA} may be configured to classify the bed rest state of the subject S into any one of coughing, body movement, respiratory abnormality, and normal when, for example, the spectral amplitude SAn_{0.2 to 0.5} (n = 1, 2, 3, 4) in the frequency band of 0.2 to 0.5 Hz and the spectral amplitude SAn_{4.6 to 5.0} (n = 1, 2, 3, 4) in the frequency band of 4.6 to 5.0 Hz are input as feature amounts (explanatory variables). Here, the respiratory abnormality includes one or more states of hypoventilation, hyperventilation, and other abnormal states related to respiration. In the following description, as an example of the respiratory abnormality, a case of "hypoventilation" being determined to be one of the bed rest states will be described.

The bed rest state of the subject S being "coughing" refers to the subject S coughing in the bed BD. The bed rest state of the subject S being "body movement" refers to the subject S exhibiting body movement in the bed BD. The "body movement" can include large body movement accompanied by movement of a torso part, small body movement not accompanied by movement of a torso part but accompanied by movement of a head part and/or four limbs, and the like. The bed rest state of the subject S being "hypoventilation" refers to the respiratory rate of the subject S on the bed BD being equal to or less than a predetermined value. The subject S is in a "hypoventilation" state, for example, when the subject S temporarily reaches an apnea state or a respiratory arrest state due to a respiratory disturbance or the like, or when the frequency of respiration decreases due to a decrease in physical strength or the like. The bed rest state of the subject S being "normal" refers to the subject S on the bed BD having not reached any of the coughing state, the body movement state, or the hypoventilation state.

Note that the bed rest state determination model M_{STA} may be capable of identifying and classifying, for example, hyperventilation as a bed rest state of the subject S, in addition to the coughing, body movement, hypoventilation, and normal states described above. The bed rest state of the subject S being "hyperventilation" refers to the respiratory rate of the subject S on the bed BD being equal to or higher than a predetermined value (however, a value different from the predetermined value when determining whether the bed rest state is "hypoventilation"). Further, the bed rest state determination model M_{STA} may identify and classify the aforementioned hypoventilation and hyperventilation, as well as other respiratory abnormality states, collectively as one category (for example, "respiratory caution") as the bed rest state of the subject S.

The bed rest state determination unit 212 sequentially inputs the parameters calculated in the preprocessing step S12 to the bed rest state determination model M_{STA} at a predetermined cycle (for example, 5 s to 15 s). The bed rest state determination model M_{STA} receives the input and outputs a determination result. The bed rest state determination unit 212 sequentially stores the output of the bed rest state determination model M_{STA} (that is, determination result) in the storage unit 22.

Note that, during a period of the subject S being away from the bed BD, the load signals ss₁ to ss₄ are not acquired and the various parameters are not calculated in the preprocessing step S12, and thus the bed rest state determination unit 212 cannot acquire the values of the parameters. When the values of the parameters cannot be acquired in a certain period, the bed rest state determination unit 212 determines the bed rest state of the subject S in the period as "bed departure".

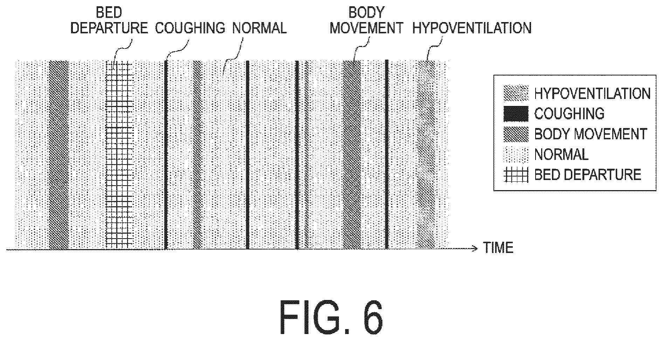

FIG. 6 is an example of a graph displaying the bed rest states of the subject S determined by the bed rest state determination unit 212 in time series. From the graph of FIG. 6, the subject S is generally understood as in a "normal" state, but occasionally reaches the "coughing" "body movement", "hypoventilation", and "bed departure" states.

### Trend Acquisition Step S14

In the trend acquisition step S14, the trend acquisition unit 213 calculates (acquires) the trend per unit time of the bed rest state of the subject S determined in the bed rest state determination step S13.

In the present embodiment, the trend of the bed rest state per unit time refers to a ratio of the bed rest state per unit time. The unit time may be set as desired and is, for example, one hour or one day. Hereinafter, the trend of the bed rest state per unit time of the subject S is referred to as a "state trend". The unit time mentioned here can be said to be synonymous with a calculation span of the trend of the bed rest state.

The trend acquisition unit 213 calculates, for each unit time elapsing, a state trend indicating a ratio of the trend of the bed rest state during the elapsed period, from the time-series data of the bed rest state during that period. The data of the state trend generated by one calculation of the trend acquisition unit 213 is counted as "(state trend of) one record" for convenience of description. However, the data of the actual state trend need not be in a record format. The trend acquisition unit 213 stores the calculated state trend of one record in the storage unit 22. Accordingly, records of the state trend are generated at the unit time interval as needed, and are stored in the storage unit 22 in time series.

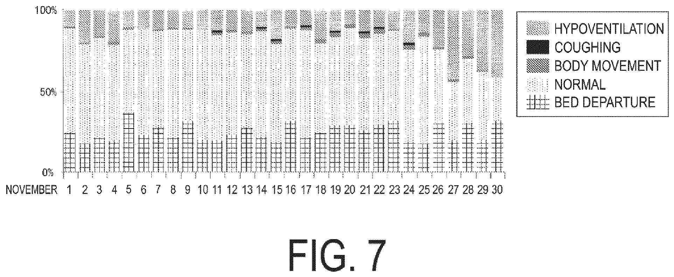

FIG. 7 is a graph obtained by arranging the state trend of the subject S in a bar graph for each record. In the example of FIG. 7, the unit time is one day, and 30 records of the state trend (that is, 30 days' worth) are arranged. Note that the graph shown in FIG. 7 is merely a schematic graph for explanation. That is, the information processing device 20 or the terminal device 30 according to the present disclosure may create or may not create a graph as illustrated in FIG. 7. In FIG. 7, one bar graph indicates a state trend (unit time = 1 day) of one record. For example, the leftmost bar graph shows the state trend in 24 hours on November 1, the state trend being approximately 25% bed departure, approximately 60% normal, approximately 2% body movement, and approximately 13% hypoventilation. Similarly, the second graph from the left shows the state trend in 24 hours on November 2, the state trend being approximately 20% bed departure, approximately 59% normal, approximately 1% body movement, and approximately 20% hypoventilation.

### Regularity Identification Step S15

In the regularity identification step S15, the regularity identification unit 214 identifies the regularity of the state trend calculated in the trend acquisition step S14, using the regularity identification model M_{REG}.

The regularity of the state trend refers to any pattern, periodicity, or the like of the state trend derived when the state trend is captured in time series. The regularity of the state trend includes, as a non-limiting example, an increase (rapid increase, gradual increase, or the like) or a decrease (rapid decrease, gradual decrease, or the like) in any one state, an increase (rapid increase, gradual increase, or the like) or a decrease (rapid decrease, gradual decrease, or the like) in any two or more states, an increase (rapid increase, gradual increase, or the like) in any one or more states and a decrease (rapid decrease, gradual decrease, or the like) in any one or more other states, or an increase (rapid increase, gradual increase, or the like) in any two or more states and a decrease (rapid decrease, gradual decrease, or the like) in any two or more other states.

The regularity identification model M_{REG} is a learned model configured to output, when two or more records of a state trend are input, regularity information including information indicating the regularity of the state trend, the information being derived from an input record group. An example of the regularity identification model M_{REG} is a learned model generated so as to identify the regularity of the state trend by pattern mining. A method for creating the regularity identification model M_{REG} will be described below.

The regularity identification unit 214 periodically inputs the state trend for a predetermined period of time (for example, least common multiple in two or more records of a state trend) to the regularity identification model M_{REG}, and identifies the regularity derived from temporal fluctuation of the state trend of the subject S. Note that the identification period of the regularity and the collection range of the records used for identification of the regularity may differ from each other. For example, when the regularity is identified every day, the records collected for regularity identification may be records from up to seven days prior to the point in time when the regularity is to be identified. The regularity identification unit 214, upon identifying the regularity, generates regularity information obtained by associating the content of the identified regularity with the date and time when the regularity is indicated, and stores the regularity information in the storage unit 22.

Here, the "date and time when the regularity is indicated" may be, for example, (i) the date and time when the regularity information is generated, (ii) the date and time of the newest record among the records of the state trend input to the regularity identification model M_{REG}, or (iii) the date and time of the oldest record among the records of the state trend input to the regularity identification model M_{REG}. Further, the "date and time" mentioned here may indicate a date, a month, a year, or the like, other than a time.

Note that, in the present embodiment, in a state of the subject S receiving care, the regularity identification unit 214 need not input records of a state trend of the ratio of "bed departure" being equal to or greater than a predetermined value (for example, about 50 to 70%) to the regularity identification model M_{REG}. With a relationship between the presence or absence of "bed departure" and the biological state of the subject S being relatively small, the regularity is identified with the state trend of a high ratio of "bed departure" being excluded, making it possible to further increase the correlation between the identified regularity and the fluctuation in the biological state of the subject S. Thus, increasing the correlation between the regularity and the fluctuation in the biological state of the subject S leads to improvement in the accuracy of the prediction of the physical condition variation prediction model M_{PRE}.

### Physical Condition Variation Prediction Step S16

In the physical condition variation prediction step S16, the physical condition variation prediction unit 215 inputs the regularity information generated in the regularity identification step S15 to the physical condition variation prediction model M_{STA}, thereby acquiring information indicating a prediction result of the physical condition variation of the subject S (hereinafter referred to as prediction information) as an output from the physical condition variation prediction model M_{STA}.

The physical condition variation prediction model M_{PRE} is a learned model created so as to predict variation in a physical condition of the subject S on the bed BD using the regularity information as an explanatory variable (creation method described below). In other words, the physical condition variation prediction model M_{PRE} is a learned model configured to output the prediction information when regularity information is input.

The variation in the physical condition of the subject S predicted by the physical condition variation prediction model M_{PRE} may be in various forms. For example, the physical condition variation prediction model M_{PRE} (i) may predict whether variation in the physical condition of the subject S will occur, (ii) may predict the probability of occurrence of variation in the physical condition of the subject S, or (iii) may predict the content of possible occurrence of variation in the physical condition of the subject S. Further, the physical condition variation prediction model M_{PRE} may predict a time of occurrence of variation in the physical condition in addition to at least one of (i) to (iii).

The physical condition variations predicted by the physical condition variation prediction model M_{PRE} of the present embodiment are, specifically, for example, fever, occurrence of attacks of various diseases such as asthma, epilepsy, and heart disease, occurrence of specific illnesses (such as pneumonia), the subject entering specific states such as hypertension, swallowing disorders, or aspiration conditions, and improvement in physical condition.

The physical condition variation prediction unit 215 sequentially stores the output of the physical condition variation prediction model M_{PRE} (that is, prediction information) in the storage unit 22.

Note that, as described above, the physical condition variation prediction model M_{PRE} "predicts" physical condition variation in the subject S. That is, the physical condition variation prediction model M_{PRE} can be said to be a model for estimating physical condition variation in the subject S at a specific time point or in a specific period in the future. Here, the "specific time point" or the "specific period" may be automatically determined on the basis of, for example, the date and time included in the regularity information. That is, on the basis of the date and time included in the input regularity information, when the physical condition variation is predicted may be determined.

Alternatively, the physical condition variation prediction model M_{PRE} may be a model configured to predict a pinpoint physical condition variation at a timing designated by date and time information using (i) regularity information and information designating a date and/or a time (hereinafter referred to as "date and time information" for convenience) as explanatory variables. Further, the physical condition variation prediction model M_{PRE} may be a model configured to predict physical condition variation for a period indicated by period information from a timing designated by the date and time information using (ii) the regularity information, the date and time information, and information designating a period (hereinafter referred to as period information) as explanatory variables. Specific examples of (i) include "predicting physical condition variation three days after the time of prediction" and "predicting physical condition variation on Apr. 1, 2023". Specific examples of (ii) include "predicting possible occurrence of physical condition variation within one month from the time of prediction".

The timing indicated by the date and time information described above may be designated or specified by the information processing device 20, or may be designated by the user of the physical condition variation prediction system 100 via the input unit 32 of the terminal device 30 or the like. In the latter case, the terminal device 30 transmits information indicating a timing (for example, date) designated by the user to the information processing device 20, and the information processing device 20 receives and uses the information.

Further, the period indicated by the period information may also be designated or specified by the information processing device 20, or may be designated by the user of the physical condition variation prediction system 100 via the input unit 32 of the terminal device 30 or the like. In this case, as in the case of the date and time information, the information indicating the predetermined period is transmitted from the terminal device 30 to the information processing device 20.

The physical condition variation prediction unit 215 acquires the prediction information as an output from the physical condition variation prediction model M_{PRE} by inputting input data required by the physical condition variation prediction model M_{PRE} in the physical condition variation prediction step S16.

### Display Step S17

In the display step S17, the control unit 21 of the information processing device 20 sequentially transmits the prediction information to the terminal device 30, thereby causing the terminal device 30 to execute a predetermined display related to the prediction of physical condition variation. The terminal device 30 performs a predetermined display on the display unit 31 on the basis of the prediction result received from the physical condition variation prediction unit 215.

Specifically, the display unit 31 displays, for example, the presence or absence of physical condition variation in the subject S indicated by the prediction result, the content of the physical condition variation, the period when the occurrence of the physical condition variation is predicted, and the like by an image and/or a sound. Further, the terminal device 30 may display at least one of the graphs shown in FIG. 5 to FIG. 7 on the display unit 31.

Note that, in the regularity identification step S15, when there is particularly no regularity derived from the record group of the state trend input to the regularity identification model M_{REG}, the regularity identification model M_{REG} may output regularity information indicating "no regularity". Further, in this case, the information processing device 20 may notify the terminal device 30 of "physical condition variation unpredictable" as the prediction information without executing the physical condition variation prediction step S16. Then, in the display step S17, the terminal device 30 may display information indicating failure to predict physical condition variation on the display unit 31.

The physical condition variation prediction method executed by the physical condition variation prediction system 100 is summarized as follows with reference to the conceptual diagram of FIG. 4. Note that the diagrams and data illustrated in each stage of FIG. 4 are merely schematic representations of data for the purpose of understanding, and the actual data need not be in such a format.

First, as illustrated in the first and second stages of FIG. 4, the load measurement unit 10 measures the analog loads si to s₄ by the load sensors LS1 to LS4, and sends the analog loads si to s₄ to the information processing device 20 (load measurement step S11). Next, as illustrated in the second and third stages of FIG. 4, the preprocessing unit 211 converts the analog loads si to s₄ into the digital loads ssi to ss₄ and calculates various parameters (preprocessing step S12).

Next, as illustrated in the third and fourth rows of FIG. 4, the bed rest state determination unit 212 inputs various parameters on the basis of the digital loads ssi to ss₄ in a predetermined period to the state determination model M_{STA}, and sequentially determines the bed rest state of the subject S at a predetermined cycle (bed rest state determination step S13). Subsequently, as illustrated in the fourth and fifth stages of FIG. 4, the trend acquisition unit 213 acquires the state trend of the bed rest state per unit time (trend acquisition step S14).

Next, as illustrated in the fifth and sixth stages of FIG. 4, the regularity identification unit 214 inputs the state trend (at least two or more records of the state trend, four records in the example of FIG. 4) in the predetermined period to the regularity identification model, and identifies the regularity (regularity identification step S15). Then, as illustrated in the sixth and seventh stages of FIG. 4, the physical condition variation identification unit 215 inputs the identified regularity (and, if necessary, date and time information and/or period information) to the physical condition variation prediction model M_{PRE}, thereby predicting the presence or absence of physical condition variation in the subject S, the probability of occurrence of the physical condition variation, the content of the physical condition variation, and/or the time of possible occurrence of the physical condition variation.

### Bed Rest State Determination Model Creation Method

Next, a method for creating the bed rest state determination model M_{STA} used in the physical condition variation prediction system 100 of the embodiment described above will be described.

The bed rest state determination model M_{STA} used in the physical condition variation prediction system 100 is created according to the content of the bed rest state to be determined.

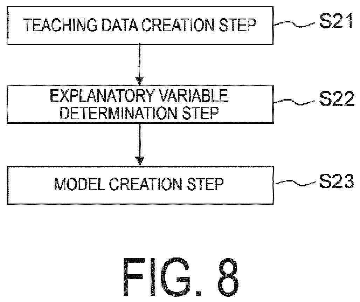

As illustrated in the flowchart of FIG. 8, the method for creating the bed rest state determination model includes a teaching data creation step S21, an explanatory variable determination step S22, and a model creation step S23.

In the teaching data creation step S21, teaching data used in the model creation step S23 is created by the following procedure as an example.

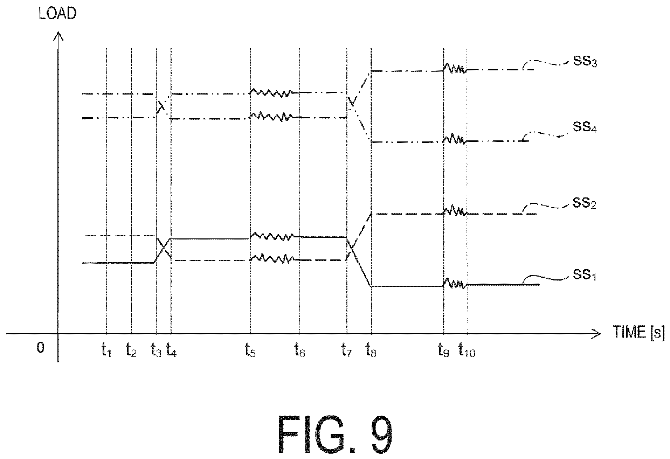

First, in a state of the subject S being present on the bed BD, measurement of the load of the subject S by the load sensors LS1 to LS4 and imaging of the subject S on the bed BD are performed in parallel over a predetermined period, and the load signals ss₁ to ss₄ and an image (moving image) of the subject S are stored. An example of the stored load signals ss₁ to ss₄ is as shown in FIG. 9. At this time, voice of the subject S may be recorded.

Next, the stored load signals ssi to ss₄ are labeled (tagged) on the basis of the stored image of the subject S. The labeling is performed by associating the types of states (motion) of the subject S and start times and end times of the states with the load signals ss₁ to ss₄.

The types of states to be labeled are, by way of example, "hypoventilation", "coughing", "normal", and "body movement". In the example shown in FIG. 9, a period from time t₁ to time t₂ is labeled as "hypoventilation", a period from time t₃ to time t₄ and a period from time t₇ to time t₈ as "body movement", a period from time t₅ to time t₆ and a period from time t₉ to time t₁₀ as "coughing", and other periods as "normal".

In place of or in addition to "hypoventilation", "coughing", "normal", and "body movement", the types of states to be labeled may include at least one of "bed entry" (movement of the subject from off the bed to on the bed), "bed departure" (movement of the subject from on the bed to off the bed), "edge sitting position" (state of the subject sitting on an edge of the bed), "sitting position" (state of the subject sitting on the bed), "angle change of reclining bed" (ascending or descending), dangerous motion on the bed (such as climbing over the bed rail), "eating", "supine", "right side lying", "left side lying", "prone", and the like.

Next, a plurality of feature amounts are created on the basis of the stored load signals ss₁ to ss₄. The feature amount used in the present embodiment may include at least one of (1) the load signals ss₁ to ss₄, (2) the spectral amplitude SAn_{X to Y} of each frequency band, (3) the position of center of gravity, (4) the respiratory waveform, (5) the respiratory rate, (6) the respiratory amplitude value, (7) the heart rate waveform, (8) the activity index (ACI), and (9) the total load described above.

As described above, a dataset is created as teaching data with a combination of a plurality of feature amounts obtained on the basis of the load signals ss₁ to ss₄ (hereinafter referred to as "feature amount set FS") as input, and the state of the subject S labeled in correspondence with the load signals ssi to ss₄ as output. With each feature amount created on the basis of the load signals ssi to ss₄, each feature amount is associated with the state of the subject S labeled in correspondence with the load signals ss₁ to ss₄.

In the explanatory variable determination step S22, at least one feature amount is selected from the feature amount set FS included in the teaching data created in the teaching data creation step S21, and is determined to be an explanatory variable to be used in the model creation step S23 described below.

As an example, the selection of the feature amount (determination of the explanatory variable) can be performed by using a gradient boosting technique (specifically, for example, XGBoost, LightGBM, or CatBoost) based on a decision tree algorithm. Note that logistic regression may be used in place of the gradient boosting technique.

Specifically, training data having the feature amount set FS as an input and a predetermined state (for example, "coughing", "body movement", "hypoventilation", or "normal") of the subject S as an output is created using the teaching data created in the teaching data creation step S21. The predetermined states include states to be classified by the created model, and at least one of a plurality of labeled states is selected. Then, using the training data, a learned model for classifying the state of the subject S into the predetermined state using the feature amount set FS as an input is created by the gradient boosting technique. Subsequently, a contribution degree of each feature amount in the created learned model is calculated on the basis of an importance degree of a decision tree algorithm, and one or more feature amounts having a high contribution degree are determined to be explanatory variables to be used in the model creation step S23.

In the model creation step S23, the bed rest state determination model M_{STA} configured to classify the state of the subject S into a predetermined state (for example, "coughing", "body movement", "hypoventilation", or "normal") is generated by supervised machine learning using the feature amounts selected in the explanatory variable determination step S22 as explanatory variables. As the supervised machine learning, specifically, for example, a naive Bayes classifier, a decision tree, a support vector machine (SVM), a neural network, a k-nearest neighbors algorithm, gradient boosting, and logistics regression can be used. The teaching data can be created by, for example, extracting the data related to the feature amount selected in the explanatory variable determination step S22 from the feature amount set FS.

### Method for Creating Regularity Identification Model

Next, the method for creating the regularity identification model M_{REG} used in the physical condition variation prediction system 100 of the embodiment described above will be described. Here, a case of the regularity identification model M_{REG} being created by using the physical condition variation prediction system 100 will be described as an example.

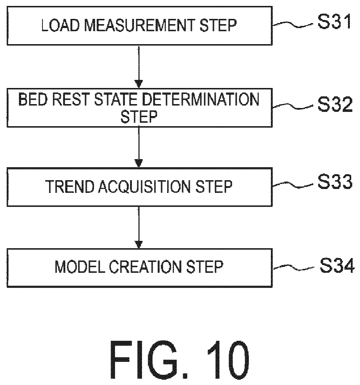

As illustrated in the flowchart of FIG. 10, the method for creating the regularity identification model M_{REG} includes a load measurement step S31, a bed rest state determination step S32, a trend acquisition step S33, and a model creation step S34.

The load measurement step S31 is the same as the load measurement step S11 in the physical condition variation prediction method. That is, in the load measurement step S31, the load measurement unit 10 detects the load of the subject S on the bed BD by the load sensors LS1 to LS4, and sends the detected load to the information processing device 20 as the analog signals s₁ to s₄.

The bed rest state determination step S32 is similar to the preprocessing step S12 and the bed rest state determination step S13 in the physical condition variation prediction method. That is, in the bed rest state determination step S32, the preprocessing unit 211 converts the analog signals s₁ to s₄ into the digital signals ssi to ss₄ and calculates various parameters on the basis of the digital signals. Then, the bed rest state determination unit 212 inputs the calculated various parameters to the bed rest state determination model M_{STA} to determine the bed rest state of the subject S.

The trend acquisition step S33 is the same as the trend acquisition step S14 in the physical condition variation prediction method. That is, in the trend acquisition step S33, the trend acquisition unit 213 calculates the trend per unit time of the bed rest state of the subject S determined by the bed rest state determination unit 212.

Note that, in the trend acquisition step S33, records having different unit times may be generated. For example, in the trend acquisition step S33, a record of the state trend may be generated every hour (that is, with the unit time set to one hour), and a record of the state trend may be generated daily with the unit time set to one day. In addition, the state trend may be calculated with the unit time being any period other than one hour or one day.

In the model creation step S34, the model creation unit 217 generates the regularity identification model M_{REG} configured to identify a predetermined regularity on the basis of one or more bed rest states by unsupervised machine learning using the trend of the bed rest state acquired in the trend acquisition step S33 as training data. As the unsupervised machine learning algorithm, specifically, for example, random forest, clustering, principal component analysis, association analysis, generative adversarial network, Gaussian mixture model (GMM), local outlier factor (LOF), isolation forest, and kernel density estimation can be used.

Note that, in the model creation step S34, it is more desirable to use a plurality of records of the state trend acquired in the trend acquisition step S33 as the training data. This makes it possible to create the regularity identification model M_{REG} capable of identifying the regularity with higher accuracy. The amount and content of the training data used in the model creation step S34 may be determined as appropriate within a range of the generalization performance of the regularity identification model M_{REG} not being compromised.

Note that, when a plurality of records of state trends are used as training data, each record may be a record group having the same specific unit time of the state trend (that is, specific span of state trend), or the unit times of some or all of the records may be different. When a record group having the same unit time is used, an identification accuracy of the regularity when the record of the unit time is input to the model can be improved. On the other hand, when a record group having different unit times is used, a model having high generalization performance can be created.

Further, in the present embodiment, among the state trends acquired in the trend acquisition step S33, a state trend having a ratio of "bed departure" being greater than or equal to a predetermined value (for example, about 50 to 70%) need not be employed as training data. With the relationship between the presence or absence of "bed departure" and the biological state of the subject S being relatively small, the training data excluding the state trend having a high ratio of "bed departure" is used, making it possible to further increase the correlation between the identified regularity and the fluctuation in the biological state of the subject S. Thus, increasing the correlation between the identified regularity and the fluctuation in the biological state of the subject S can lead to improvement in the accuracy of the prediction of the physical condition variation prediction model M_{PRE}.

Herein, the regularities of the state trend to be identified by the regularity identification model M_{REG} are, for example, the following regularities.
(1) The ratio of "coughing" increases every day for two or more days.
(2) At a predetermined timing (for example, every day, after 17 o' clock in the evening), the ratio of "coughing" continues to increase for three days or more.
(3) The ratio of "hypoventilation" decreases for three or more consecutive days, and the ratio of "normal" increases for three or more consecutive days.
(4) The ratio of "hypoventilation" increases every day for 25 days or more.
(5) The ratio of "hypoventilation" decreases every day, and the ratio of "body movement" increases every day and continues for one week or more.

These regularities are examples of regularities having a correlation with physical condition variation in the subject, and regularity information indicating these regularities is included in the teaching data as explanatory variables in a method for creating a physical condition variation prediction model described below.

### Method for Creating Physical Condition Variation Prediction Model

Next, the method for creating the physical condition variation prediction model M_{PRE} used in the physical condition variation prediction system 100 of the embodiment described above will be described. Here, a case of the information processing device 20 included in the system for predicting physical condition variation (that is, physical condition variation prediction system 100) described above creating the physical condition variation prediction model M_{PRE} will be described as an example.

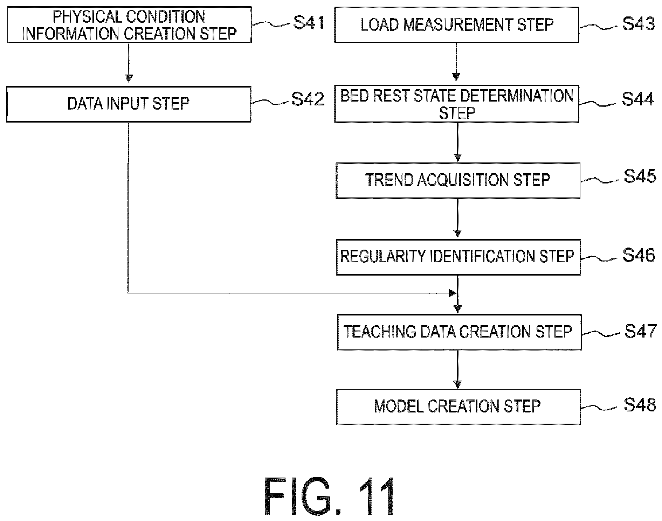

As illustrated in the flowchart of FIG. 11, the method for creating the physical condition variation prediction model M_{PRE} includes a physical condition information creation step S41, a data input step 42, a load measurement step S43, a bed rest state determination step S44, a trend acquisition step S45, a regularity identification step S46, a teaching data creation step S47, and a model creation step S48.

In the physical condition information creation step S41, the information processing device 20 creates or acquires physical condition information indicating the physical condition of the subject S in time series. Specifically, the physical condition information is created by, for example, a data creator such as a doctor, a nurse, or a caregiver visually observing the physical condition of the subject S and recording the observation result as electronic data in the information processing device 20, the terminal device 30, another device, or the like. A nursing record created by a nurse in a hospital or the like, a caregiving record created by a caregiver in a caregiving facility, and the like are examples of the physical condition information. The information processing device 20 creates the physical condition information on the basis of the observation result input to the information processing device 20. Alternatively, the information processing device 20 acquires the physical condition information by acquiring the physical condition information from the terminal device 30 or another device periodically or at a predetermined timing.

Instead of or in addition to the data creation by the data creator through visual observation, the biological information of the subject S may be measured using various sensors, the measured biological information may be recorded in the information processing device 20, the terminal device 30, another device, or the like together with the measurement date and time, and the recorded data may be used as the physical condition information. Examples of the various sensors include a thermometer and a pulsimeter configured to measure vital data such as pulse, blood pressure, respiration, and body temperature.

The created physical condition information is data associating the physical condition of the subject S and the time with each other. Accordingly, it is possible to identify the time and the state of the physical condition of the subject S on the basis of the physical condition information. Note that the "time" mentioned here is not limited to the time of one day, and temporal divisions are not particularly limited as long as the divisions indicate periods such as dates, months, or years.

The physical conditions of the subject S includable in the physical condition information specifically include, for example, heart rate, respiratory rate, blood pressure, body temperature, and blood oxygen concentration.

Further, in the present embodiment, the physical condition information also includes behavioral information of the subject S, such as eating and body cleansing of the subject S. This behavioral information may be included in the nursing record and/or the caregiving record described above. In the model creation step S48 described below, by performing learning in consideration of the effects of fluctuation in the load according to the behavior of the subject S, it is possible to enhance the prediction accuracy of the created physical condition variation prediction model M_{PRE}.

In the data input step S42, the physical condition information created in the physical condition information creation step S41 is input to the information processing device 20. The physical condition information can be input to the information processing device 20 via, for example, the input unit 32 of the terminal device 30. In addition, the input may be performed by transmitting the physical condition information stored in an external device such as a personal computer (PC) to the information processing device 20. The information processing device 20 stores the received physical condition information in the storage unit 22.

The load measurement step S43 is the same as the load measurement step S11 in the physical condition variation prediction method. That is, in the load measurement step S43, the load measurement unit 10 detects the load of the subject S on the bed BD by the load sensors LS1 to LS4, and sends the detected load to the information processing device 20 as the analog signals s₁ to s₄.

The bed rest state determination step S44 is similar to the preprocessing step S12 and the bed rest state determination step S13 in the physical condition variation prediction method. That is, in the bed rest state determination step S44, the preprocessing unit 211 converts the analog signals s₁ to s₄ into the load signals ssi to ss₄, and calculates various parameters on the basis of the load signals. Then, the bed rest state determination unit 212 inputs the calculated various parameters to the bed rest state determination model M_{STA} to determine the bed rest state of the subject S.

The trend acquisition step S45 is the same as the trend acquisition step S14 in the physical condition variation prediction method. That is, in the trend acquisition step S45, the trend acquisition unit 213 acquires the trend per unit time of the bed rest state of the subject S determined by the bed rest state determination unit 212.

The regularity identification step S46 is the same as the regularity identification step S15 in the physical condition variation prediction method. That is, in the regularity identification step S46, the regularity identification unit 214 identifies the regularity included in the temporal fluctuation of the state trend by using the regularity identification model M_{REG}. When the regularity is identified, the regularity identification unit 214 generates the regularity information and stores the regularity information in the storage unit 22. Further, in the regularity identification step S46, the regularity identification model M_{REG} uses, as the "regularity information", information obtained by associating the content of the regularity identified by the model with the date and time when the regularity is indicated. The "date and time when the regularity is indicated" may be, for example, (i) the date and time when the regularity information is generated, (ii) the date and time of the newest record among the records of the state trend input to the regularity identification model M_{REG}, or (iii) the date and time of the oldest record among the records of the state trend input to the regularity identification model M_{REG}. Further, the "date and time" mentioned here may indicate a date, a month, a year, or the like, other than a time.

In the teaching data creation step S47, the teaching data creation unit 217 creates teaching data by using the physical condition information stored in the storage unit 22 in the data input step S42 and the regularity information stored in the storage unit 22 in the regularity identification step S46.

The created teaching data is a dataset with the regularity of the state trend of the subject S as an input and the physical condition information of the subject S as an output. With both the regularity information and the physical condition information being associated with the date and time, by combining certain regularity information and physical condition information indicating a date and time within a predetermined period from the date and time indicated by the certain regularity information, for example, it is possible to create one set of teaching data indicating "how the actual physical condition transitioned when the state trend up to a specific date and time had a certain regularity". Note that, when there are a plurality of physical condition information items indicating dates and times within a predetermined period from a date and time indicated by certain regularity information, a plurality of teaching data items may be created from the certain regularity information. That is, teaching data obtained by combining certain regularity information and first physical condition information and teaching data obtained by combining the same regularity information and second physical condition information may be created. In the created teaching data, the regularity of the state trend and the physical condition of the subject S are associated with each other via the date and time.

In the model creation step S48, the model creation unit 218 causes the prediction model to learn correlations between the regularity of the state trend of the subject S and the physical condition information of the subject S by supervised machine learning using the teaching data created in the teaching data creation step S47. Accordingly, the physical condition variation prediction model M_{PRE} predicting the physical condition variation of the subject S on the bed BD is generated on the basis of the regularity of the state trend. As the supervised machine learning, specifically, for example, a naive Bayes classifier, a decision tree, a support vector machine (SVM), a neural network, k-nearest neighbors algorithm, gradient boosting, and logistics regression can be used.

The reason it is possible to create the physical condition variation prediction model M_{PRE} by causing the prediction model to learn the correlation between the regularity of the state trend of the subject S and the physical condition information of the subject S is as follows.

According to the findings of the inventors of the present invention, before any variation occurs in the physical condition of the subject S, some regular fluctuation often occurs in the bed rest state of the subject S. As a specific example, the following relationship has been observed. Note that the number of days in the following example is described by using, as a starting point, the acquisition date of the record of the state trend serving as a starting point for identifying regularity.

(1) When the ratio of "coughing" continues to increase every day for two days or more, fever occurs after four days.
(2) When the ratio of "coughing" increases at a predetermined timing (for example, 17 o' clock in the evening) for three days or more, fever occurs after three days and 12 hours.
(3) In a subject having a high "hypoventilation" ratio, when the"hypoventilation" ratio decreases and the "normal" ratio increases, then fever occurs.
(4) In a subject having a high "hypoventilation" ratio, when the "hypoventilation" ratio continues to increase every day for 25 days or more, the subject passes away after one month.
(5) In a subject having a high "normal" ratio, when the"hypoventilation" ratio continues to decrease every day and the "body movement" ratio continues to increase every day for one week or more, the physical condition improves from the eighth day.

Thus, a correlation exists between the fluctuation in the bed rest state of the subject S and the variation in the physical condition of the subject. Accordingly, by causing the prediction model to learn the correlation between the fluctuation in the bed rest state of the subject S and the variation in the physical condition of the subject, it is possible to create a prediction model configured to predict the variation in the physical condition of the subject on the basis of the fluctuation in the bed rest state of the subject. Further, by using, as the input to the prediction model, the regularity of temporal fluctuation of the state trend of the bed rest state of the subject S rather than temporal fluctuation of the bed rest state of the subject S itself, it is possible to perform more accurate prediction obtained by suppressing the effects of irregular fluctuation in the bed rest state of the subject S.

As described above, the physical condition variation prediction system 100 and the physical condition variation prediction method of the embodiment described above predict physical condition variation in the subject S by using the physical condition variation prediction model M_{PRE} with the temporal fluctuation in the state trend of the bed rest state of the subject S as an input. Accordingly, it is possible to predict variation in the physical condition of the subject S with high accuracy by suppressing the effects of irregular fluctuation in the bed rest state of the subject S. Further, in the method for creating a physical condition variation prediction model of the embodiment described above, by causing the prediction model to learn the correlation between the regularity of the state trend of the subject S and the physical condition information of the subject S, it is possible to create a highly accurate physical condition variation prediction model obtained by suppressing the effects of the irregular fluctuation of the bed rest state of the subject S.

In the embodiment described above, the following modified aspects can also be used.

In the embodiment described above, the bed rest state determination model M_{STA}, the regularity identification model M_{REG}, and the physical condition variation prediction model M_{PRE} are created by using various data on a certain subject (subject S). That is, in the embodiment described above, a case of the subject (subject S) of physical condition variation prediction and the subject for the teaching data of the bed rest state determination model M_{STA} as well as the physical condition variation prediction model M_{PRE} being the same has been described. Further, the records of the state trend input during creation of the regularity identification model M_{REG} are also those of the subject S. In other words, in the embodiment described above, the bed rest state determination model M_{STA}, the regularity identification model M_{REG}, and the physical condition variation prediction model M_{PRE} are each a learned model obtained by learning an autocorrelation of a plurality of parameters related to the subject S.

However, the method for creating the bed rest state determination model M_{STA}, the regularity identification model M_{REG}, and the physical condition variation prediction model M_{PRE} according to the present disclosure is not limited to this method. For example, one or more models among the three types of models may be created from data on a subject (or a subject group) other than the subject (subject S) of physical condition variation prediction.

That is, each of the bed rest state determination model M_{STA}, the regularity identification model M_{REG}, and the physical condition variation prediction model M_{PRE} may be a learned model obtained by learning the correlation between a plurality of parameters obtained from the other subject (or subject group) described above. In other words, a subject of the collection of data (for example, load data or physical condition information) necessary for creating various learned models may be different from the actual subject of physical condition variation prediction.

Further, the bed rest state determination model M_{STA}, the regularity identification model M_{REG}, and the physical condition variation prediction model M_{PRE} may be created by using both data related to the subject (subject S) of physical condition variation prediction and data obtained from another subject (or subject group). That is, the bed rest state determination model M_{STA}, the regularity identification model M_{REG}, and the physical condition variation prediction model M_{PRE} may each be a learned model obtained by learning the correlation of parameters related to a plurality of persons including the subject S.

In the embodiment described above, the creation of the bed rest state determination model M_{STA}, the creation of the regularity identification model M_{REG}, the creation of the physical condition variation prediction model M_{PRE}, and the prediction of the physical condition variation are all performed by the physical condition variation prediction system 100 illustrated in FIG. 1. However, the physical condition variation prediction system 100 may be a system configured to predict physical condition variation by using the bed rest state determination model M_{STA}, the regularity identification model M_{REG}, and the physical condition variation prediction model M_{PRE} created in advance. In this case, the information processing device 20 of the physical condition variation prediction system 100 need not include the teaching data creation unit 216 and the model creation unit 217.

Further, when various learned models (bed rest state determination model M_{STA}, regularity identification model M_{REG}, and physical condition variation prediction model M_{PRE)} are created by a system and a device other than the physical condition variation prediction system 100, the same configuration as the configuration of the physical condition variation prediction system 100 is not necessarily essential.

For example, when the bed rest state determination model M_{STA} is created, the bed rest state determination model M_{STA} can be created by using the load measurement unit 10 and a model creation device such as a computer capable of communicating with the load measurement unit 10. In this case, a control unit of the model creation device corresponds to the control unit 21, and the control unit of the model creation device includes at least the preprocessing unit 211 and the bed rest state determination unit 212. Further, an unlearned model serving as a template of the bed rest state determination model M_{STA} may be stored in a storage unit of the computer or an external storage device capable of communicating with the computer.

Further, when the regularity identification model M_{REG} is created, for example, the regularity identification model M_{REG} can be created by using the load measurement unit 10, a model creation device such as a computer capable of communicating with the load measurement unit 10, and the created bed rest state determination model M_{STA}. In this case, a control unit of the model creation device corresponds to the control unit 21, and the control unit of the model creation device includes at least the preprocessing unit 211, the bed rest state determination unit 212, the trend acquisition unit 213, and the regularity identification unit 214. Further, the created bed rest state determination model M_{STA} is stored in a storage unit of the computer or an external storage device capable of communicating with the computer, and the control unit of the model creation device determines the bed rest state using this model. Note that the storage unit or the storage device may store an unlearned model serving as a template of the regularity identification model M_{REG}.

Further, when the physical condition variation prediction model M_{PRE} is created, for example, the physical condition variation prediction model M_{PRE} can be created by using the load measurement unit 10, a device such as the terminal device 30 serving as an acquisition destination of the physical condition information, a model creation device such as a computer capable of communicating with these two devices, the created bed rest state determination model M_{STA}, and the created regularity identification model M_{REG}. In this case, a control unit of the model creation device corresponds to the control unit 21, and the control unit of the model creation device includes at least the preprocessing unit 211, the bed rest state determination unit 212, the trend acquisition unit 213, the regularity identification unit 214, and the physical condition variation prediction unit 215. Further, the created bed rest state determination model M_{STA} and the created regularity identification model M_{REG} are stored in a storage unit of the computer or an external storage device capable of communicating with the computer, and the control unit of the model creation device determines the bed rest state and identifies the regularity using these models. Note that the storage unit or the storage device may store an unlearned model serving as a template of the physical condition variation prediction model MPRE.

Two or more of the creation of the three learned models and the prediction of the physical condition variation described above may be collectively implemented by the same system. For example, the bed rest state determination model M_{STA} and the regularity identification model M_{REG} may be created in advance by the same model creation device, and the created models may be stored in the storage unit 22 of the information processing device 20 of the physical condition variation prediction system 100. Then, in the physical condition variation prediction system 100, the creation of the physical condition variation prediction model M_{PRE} and the prediction of the physical condition variation may be executed.

In the physical condition variation prediction method of the embodiment described above, the display step S17 may be omitted. In this case, the user of the physical condition variation prediction system 100 may periodically check whether information (prediction information) indicating the prediction result of the physical condition variation is stored in the storage unit 22 of the information processing device 20. When the information indicating the prediction result of the physical condition variation (prediction information) is stored, the user can perform, on the subject S, a preventive treatment corresponding to the content of the information.

In the teaching data generation step S47 of the physical condition variation model creation method of the embodiment described above, data obtained by associating, through time, the regularity of the state trend of the subject S and environmental information such as a temperature and a humidity of a hospital room or the like provided with the bed BD may be included as teaching data. Then, the teaching data may be used in the model creation step S48. The surrounding environment of the subject S directly or indirectly affects the physical condition (body temperature, respiratory rate, and the like) of the subject S. Accordingly, by causing the prediction model to learn the correlation between the bed rest state of the subject S and the surrounding environment of the subject S, it is possible to further improve the prediction accuracy of the physical condition variation prediction model created.

In the physical condition variation model creation method according to the embodiment described above, a case of creating and using teaching data being by using the physical condition information and the regularity of the state trend of the subject S himself/herself of the prediction of the physical condition variation has been described. However, the regularity information and/or the physical condition information used as the teaching data is not limited to the information of the subject S himself/herself. For example, in addition to or instead of the teaching data based on the subject S himself/herself of the physical condition variation prediction, one or more teaching data items based on one or more subjects different from the subject S of the physical condition variation prediction may be created and used.

In the method for creating a physical condition variation prediction model according to the embodiment described above, the behavioral information of the subject S such as the eating and the body cleansing of the subject S is included in the physical condition information of the subject S, but the method is not limited to this inclusion. The physical condition information need not include the behavioral information.

Further, the physical conditions included in the physical condition information may be only the physical condition variation to be predicted by the physical condition variation prediction model M_{PRE} created. Even with such teaching data, the correlation between the regularity of the state trend of the subject S and the state variation of the subject S to be predicted can be learned by the prediction model. In the present invention, data indicating the correspondence between the physical condition variation to be predicted and the time included in the physical condition information is referred to as "physical condition variation information".

In the physical condition variation prediction system 100 described above, the physical condition variation prediction method, and the method for creating a physical condition variation prediction model, the regularity of the temporal fluctuation in the state trend of the subject S is identified by using the learned regularity identification model M_{REG}. However, the method for identifying the regularity of the state trend of the subject S is not limited to this method. For example, the regularity can be identified by any technique for identifying a pattern in data, such as pattern matching.

In the physical condition variation prediction system 100 of the embodiment described above, the load measurement unit 10 may measure the load of the subject S by a plurality of pressure-sensitive sensors (pressure sensors) disposed in a matrix under a sheet in place of the load sensors LS1 to LS4.

The physical condition variation prediction system 100 of the embodiment described above does not necessarily need to include all of the load sensors LS1 to LS4, and may include only any one of these sensors. The load detectors do not necessarily need to be positioned at the four corners of the bed and can be disposed at any position so that the load of the subject on the bed and the fluctuation of the load can be detected. Further, the load sensors LS1 to LS4 are not limited to load sensors using load cells having a beam shape and, for example, force sensors can also be used.

In the physical condition variation prediction system 100 of the embodiment described above, a camera, an acceleration sensor, or the like configured to acquire the biological information of the subject on the bed BD may be used instead of or in addition to the load measurement unit 10. In the case of acquiring image information of the subject S as the biological information by using a camera, or in the case of acquiring the body movement of the subject S as the biological information by using an acceleration sensor attached to the subject S as well, it is possible to predict physical condition variation in the subject S by a method substantially similar to the method when the load of the subject S is acquired as the biological information.

For example, various techniques capable of identifying a motion carried out by a person in an image captured by a camera by analyzing the image exist in the related art. With utilization of such known techniques, by imaging the bed BD (and subject S) with a camera and analyzing the image, for example, it is possible to identify the motion of the subject S on the bed BD (that is, bed rest state described in the present disclosure). Note that, when the bed BD is imaged by the camera and the subject S is not visible, the subject S need only be determined to be in the "bed departure" state. The processing after the bed rest state is identified is similar to the processing described in the embodiment described above.

Further, for example, by causing the subject S to wear a wearable device equipped with an acceleration sensor, it is possible to identify the bed rest state of the subject S lying on the bed BD. The processing after the bed rest state is identified is similar to the processing described in the embodiment described above.

The physical condition variation prediction system 100 of the embodiment described above need not include the terminal device 30. Further, the information processing device 20 is not limited to a mode of being connected to the load measurement unit 10 via the network, and may be disposed in the vicinity of the load measurement unit 10 and the bed BD (for example, in the same room) and connected to the load measurement unit 10 by a wiring line.

The bed rest state determination model M_{STA}, the regularity identification model M_{REG}, and the physical condition variation prediction model M_{PRE} of the embodiment described above may be recorded on a non-transitory computer-readable recording medium. By reading the recording medium with a computer such as the information processing device 20, the computer can be caused to execute the bed rest state determination step S13, the regularity identification step S15, and the physical condition variation prediction step S16. That is, the learned models such as the bed rest state determination model M_{STA}, the regularity identification model M_{REG}, and the physical condition variation prediction model M_{PRE} can be recorded as a program in a recording medium. Further, when unlearned models serving as templates of these learned models exist, the unlearned models may also be recorded in a non-transitory and computer-readable recording medium.

As long as the features of the present invention are maintained, the present invention is not limited to the embodiment described above, and other forms considered within the scope of the technical concept of the present invention are also included within the scope of the present invention.

### Reference Signs List

10 Load measurement unit; 20 Information processing device; 211 Preprocessing unit; 212 Bed rest state determination unit; 213 Trend acquisition unit; 214 Regularity identification unit; 215 Physical condition variation prediction unit; 22 Storage unit; 30 Terminal device; BD Bed; LS1 to LS4 Load sensor; M_{PRE} Physical condition variation prediction model; M_{REG} Regularity identification model; M_{STA} Bed rest state determination model; S Subject

## Claims

1. A method for creating a physical condition variation prediction model for predicting variation in a physical condition of a subject on a bed, the method comprising:
acquiring biological information of a subject for teaching data by a sensor;
determining a bed rest state of the subject for teaching data on the basis of the biological information of the subject for teaching data;
acquiring a state trend corresponding to a trend of the bed rest state per unit time;
identifying a regularity included in temporal fluctuation of the state trend; and
creating, by supervised machine learning using teaching data obtained by associating the regularity and physical condition variation information indicating variation in a physical condition of the subject for teaching data, a physical condition variation prediction model obtained by learning a correlation between the regularity and the physical condition variation information of the subject for teaching data.

2. The method according to claim 1, wherein
the subject and the subject for teaching data are the same.

3. The method according to claim 1, wherein
the subject and the subject for teaching data are different.

4. The method according to any one of claims 1 to 3, wherein
the physical condition variation information includes presence or absence of variation in the physical condition of the subject for teaching data and/or content of the variation in the physical condition of the subject for teaching data.

5. The method according to any one of claims 1 to 4, wherein
the bed rest state includes a coughing state, a body movement state, a normal state, and a respiratory abnormality state.

6. The method according to any one of claims 1 to 5, wherein
the bed rest state includes a bed departure state, and
in the identifying the regularity included in the temporal fluctuation of the state trend, the regularity included in the temporal fluctuation of the state trend, excluding a state trend of a ratio of the bed departure state being equal to or greater than a predetermined value, is identified.

7. The method according to any one of claims 1 to 6, wherein
the teaching data is data obtained by further associating the regularity and behavioral information indicating a behavior of the subject for teaching data and/or environmental information indicating an environment surrounding the subject for teaching data.

8. The method according to any one of claims 1 to 7, wherein
the identifying the regularity included in the temporal fluctuation of the state trend is performed by a learned regularity identification model learned so as to output the regularity when the state trend is input.

9. The method according to any one of claims 1 to 8, wherein
the sensor is a load sensor disposed on the bed, and
the biological information is a load of the subject for teaching data.

10. The method according to claim 9, wherein
the load sensor includes four sensors disposed at four corners of the bed, respectively, and
each of the four sensors includes
a strain body configured to generate a strain corresponding to the load of the subject for teaching data, and
a strain sensor attached to the strain body.

11. A physical condition variation prediction system for predicting variation in a physical condition of a subject on a bed, the physical condition variation prediction system comprising:
a bed rest state determination unit configured to determine a bed rest state of the subject on the basis of biological information of the subject acquired by a sensor;
a state trend acquisition unit configured to acquire a state trend corresponding to a trend of the bed rest state per unit time;
a regularity identification unit configured to identify a regularity included in temporal fluctuation of the state trend; and
a physical condition variation prediction unit configured to input the regularity into a physical condition variation prediction model to predict variation in a physical condition of the subject, wherein
the physical condition variation prediction model is a learned model obtained by learning, by supervised machine learning using teaching data obtained by associating the regularity identified for a subject for teaching data and physical condition variation information indicating variation in a physical condition of the subject for teaching data, a correlation between the regularity and the physical condition variation information of the subject for teaching data.

12. The physical condition variation prediction system according to claim 11, wherein
the subject and the subject for teaching data are the same.

13. The physical condition variation prediction system according to claim 11, wherein
the subject and the subject for teaching data are different.

14. The physical condition variation prediction system according to any one of claims 11 to 13, wherein
the physical condition variation information includes presence or absence of variation in the physical condition of the subject for teaching data and/or content of the variation in the physical condition of the subject for teaching data.

15. The physical condition variation prediction system according to any one of claims 11 to 14, wherein
the bed rest state includes a coughing state, a body movement state, a normal state, and a respiratory abnormality state.

16. The physical condition variation prediction system according to any one of claims 11 to 15, wherein
the bed rest state includes a bed departure state, and
in the identifying the regularity included in the temporal fluctuation of the state trend, the regularity included in the temporal fluctuation of the state trend, excluding a state trend of a ratio of the bed departure state being equal to or greater than a predetermined value, is identified.

17. The physical condition variation prediction system according to any one of claims 11 to 16, wherein
the teaching data is data obtained by further associating the regularity and behavioral information indicating a behavior of the subject for teaching data and/or environmental information indicating an environment surrounding the subject for teaching data.

18. The physical condition variation prediction system according to any one of claims 11 to 17, wherein the regularity identification unit identifies the regularity included in the temporal fluctuation of the state trend by a learned regularity identification model learned so as to output the regularity when the state trend is input.

19. The physical condition variation prediction system according to any one of claims 11 to 18, wherein
the sensor is a load sensor disposed on the bed, and
the biological information is a load of the subject.

20. The physical condition variation prediction system according to claim 19, wherein
the load sensor includes four sensors disposed at four corners of the bed, respectively, and
each of the four sensors includes
a strain body configured to generate a strain corresponding to the load of the subject, and
a strain sensor attached to the strain body.

21. A physical condition variation prediction method for predicting variation in a physical condition of a subject on a bed, the physical condition variation prediction method comprising:
determining a bed rest state of the subject on the basis of biological information of the subject acquired by a sensor;
acquiring a state trend corresponding to a trend of the bed rest state per unit time;
identifying a regularity included in temporal fluctuation of the state trend; and
inputting the regularity into a physical condition variation prediction model to predict variation in a physical condition of the subject, wherein
the physical condition variation prediction model is a learned model obtained by learning, by supervised machine learning using teaching data obtained by associating the regularity identified for a subject for teaching data and physical condition variation information indicating variation in a physical condition of the subject for teaching data, a correlation between the regularity and the physical condition variation information of the subject for teaching data.

22. The physical condition variation prediction method according to claim 21, wherein
the subject and the subject for teaching data are the same.

23. The physical condition variation prediction method according to claim 21, wherein
the subject and the subject for teaching data are different.

24. The physical condition variation prediction method according to any one of claims 21 to 23, wherein
the physical condition variation information includes presence or absence of variation in the physical condition of the subject for teaching data and/or content of the variation in the physical condition of the subject for teaching data.

25. The physical condition variation prediction method according to any one of claims 21 to 24, wherein
the bed rest state includes a coughing state, a body movement state, a normal state, and a respiratory abnormality state.

26. The physical condition variation prediction method according to any one of claims 21 to 25, wherein
the bed rest state includes a bed departure state, and
in the identifying the regularity included in the temporal fluctuation of the state trend, the regularity included in the temporal fluctuation of the state trend, excluding a state trend of a ratio of the bed departure state being equal to or greater than a predetermined value, is identified.

27. The physical condition variation prediction method according to any one of claims 21 to 26, wherein
the teaching data is data obtained by further associating the regularity and behavioral information indicating a behavior of the subject for teaching data and/or environmental information indicating an environment surrounding the subject for teaching data.

28. The physical condition variation prediction method according to any one of claims 21 to 27, wherein
in the identifying the regularity, the identifying the regularity identifies the regularity included in the temporal fluctuation of the state trend by a learned regularity identification model learned so as to output the regularity when the state trend is input.

29. The physical condition variation prediction method according to any one of claims 21 to 28, wherein
the sensor is a load sensor disposed on the bed, and
the biological information is a load of the subject.

30. The physical condition variation prediction method according to claim 29, wherein
the load sensor includes four sensors disposed at four corners of the bed, respectively, and
each of the four sensors includes
a strain body configured to generate a strain corresponding to the load of the subject, and
a strain sensor attached to the strain body.

31. A physical condition variation prediction model for predicting variation in a physical condition of a subject on a bed, wherein
by supervised machine learning using teaching data created by
acquiring biological information of a subject for teaching data by a sensor,
determining a bed rest state of the subject for teaching data on the basis of the biological information of the subject for teaching data,
acquiring a state trend corresponding to a trend of the bed rest state per unit time,
identifying a regularity included in temporal fluctuation of the state trend, and
associating the regularity and physical condition variation information indicating variation in a physical condition of the subject for teaching data,
a correlation between the regularity and the physical condition variation information of the subject for teaching data is learned, and
variation in a physical condition of the subject is predicted on the basis of the regularity.

32. The physical condition variation prediction model according to claim 31, wherein
the subject and the subject for teaching data are the same.

33. The physical condition variation prediction model according to claim 31, wherein
the subject and the subject for teaching data are different.

34. The physical condition variation prediction model according to any one of claims 31 to 33, wherein
the physical condition variation information includes presence or absence of variation in the physical condition of the subject for teaching data and/or content of the variation in the physical condition of the subject for teaching data.

35. The physical condition variation prediction model according to any one of claims 31 to 34, wherein
the bed rest state includes a coughing state, a body movement state, a normal state, and a respiratory abnormality state.

36. The physical condition variation prediction model according to any one of claims 31 to 35, wherein
the bed rest state includes a bed departure state, and
in the identifying the regularity included in the temporal fluctuation of the state trend, the regularity included in the temporal fluctuation of the state trend, excluding a state trend of a ratio of the bed departure state being equal to or greater than a predetermined value, is identified.

37. The physical condition variation prediction model according to any one of claims 31 to 36, wherein
the teaching data is data obtained by further associating the regularity and behavioral information indicating a behavior of the subject for teaching data and/or environmental information indicating an environment surrounding the subject for teaching data.

38. The physical condition variation prediction model according to any one of claims 31 to 37, wherein
the identifying the regularity included in the temporal fluctuation of the state trend is performed by a learned regularity identification model learned so as to output the regularity when the state trend is input.

39. The physical condition variation prediction model according to any one of claims 31 to 38, wherein
the sensor is a load sensor disposed on the bed, and
the biological information is a load of the subject for teaching data.

40. The physical condition variation prediction model according to claim 39, wherein
the load sensor includes four sensors disposed at four corners of the bed, respectively, and
each of the four sensors includes
a strain body configured to generate a strain corresponding to the load of the subject for teaching data, and
a strain sensor attached to the strain body.

41. A non-transitory computer-readable recording medium for storing the physical condition variation prediction model according to any one of claims 31 to 40.

42. A method for creating a regularity identification model, the method comprising:
acquiring biological information of a subject on a bed by a sensor;
determining a bed rest state of the subject on the basis of the biological information of the subject;
acquiring a state trend corresponding to a trend of the bed rest state per unit time; and
creating, by machine learning using the state trend as training data, a regularity identification model configured to identify a predetermined regularity included in temporal fluctuation of the state trend.
